# EUROPEAN PATENT SPECIFICATION

(11) **EP 3 312 294 B1**
(45) Date of publication and mention of the grant of the patent: **26.02.2020**
(21) Application number: 17001719.8
(22) Date of filing: 18.10.2017
(51) Int. Cl.: C12Q 1/689, C12Q 1/682

(54) **LOOPED UVEX TYPE PROBES AND PRIMERS FOR THE DETECTION OF TICK-TRANSMITTED PATHOGENS AND USE THEREOF IN OPTIMIZED PCR REACTION IN ORDER TO IDENTIFY AN AMPLIFIED GENETIC MATERIAL**
SCHLAUFENFÖRMIGE UVEX-SONDEN UND PRIMER ZUM NACHWEIS VON DURCH ZECKEN ÜBERTRAGENEN PATHOGENEN UND VERWENDUNG DAVON IN EINER OPTIMIERTEN PCR-REAKTION ZUR IDENTIFIZIERUNG VON AMPLIFIZIERTEM GENETISCHEN MATERIAL
SONDES DE TYPE UVEX EN BOUCLE ET AMORCES POUR LA DÉTECTION DE PATHOGÈNES TRANSMIS PAR DES TIQUES ET LEUR UTILISATION DANS UNE RÉACTION PCR OPTIMISÉE AFIN D'IDENTIFIER UN MATÉRIAU GÉNÉTIQUE AMPLIFIÉ

(30) Priority: 18.10.2016 PL 41915916
(43) Date of publication of application: 25.04.2018
(73) Proprietor: Uniwersytet Gdanski, 80-309 Gdansk (PL)
(72) Inventor: Wegrzyn, Grzegorz, 80-299 Gdansk (PL); Nejman-Falenczyk, Bozena, 80-298 Gdansk (PL); Bloch, Sylwia, 80-502 Gdansk (PL)
(74) Representative: Pawlowska, Justyna

(56) References cited:
- WO-A2-2015/038634
- KAMFAI CHAN ET AL: "Sensitive multiplex PCR assay to differentiate Lyme spirochetes and emerging pathogens Anaplasma phagocytophilum and Babesia microti", BMC MICROBIOLOGY, BIOMED CENTRAL LTD, GB, vol. 13, no. 1, 20 December 2013 (2013-12-20), page 295, XP021172032, ISSN: 1471-2180, DOI: 10.1186/1471-2180-13-295
- BOZENA NEJMAN-FALENCZYK ET AL: "A Simple and Rapid Procedure for the Detection of Genes Encoding Shiga Toxins and Other Specific DNA Sequences", TOXINS, vol. 7, no. 12, 13 November 2015 (2015-11-13), pages 4745-4757, XP55455462, DOI: 10.3390/toxins7114745
- JIANWEI JEFFERY LI ET AL: "Enzymatic signal amplification of molecular beacons for sensitive DNA detection", NUCLEIC ACIDS RESEARCH, vol. 36, no. 6, 27 February 2008 (2008-02-27), page e36, XP055455636, ISSN: 0305-1048, DOI: 10.1093/nar/gkn033
- JING ZHENG ET AL: "Rationally designed molecular beacons for bioanalytical and biomedical applications", CHEMICAL SOCIETY REVIEWS, vol. 44, no. 10, 1 January 2015 (2015-01-01), pages 3036-3055, XP055455375, ISSN: 0306-0012, DOI: 10.1039/C5CS00020C
- SU-XIA HAN ET AL: "Molecular Beacons: A Novel Optical Diagnostic Tool", ARCHIVUM IMMUNOLOGIAE ET THERAPIAE EXPERIMENTALIS, vol. 61, no. 2, 5 January 2013 (2013-01-05), pages 139-148, XP055455379, CH ISSN: 0004-069X, DOI: 10.1007/s00005-012-0209-7

## Description

The scope of the invention is defined by the appended claims. The object of the invention is the Looped UVEx type probe and its application, together with an appropriately designed pair of primers, in optimized Polymerase Chain Reaction (PCR) with Hot Start *Taq* DNA Polymerase in order to: (i) enable quick detection of the PCR product by visual observation of color change of the post PCR mixture, directly in the reaction tube, under UV light; (ii) increase the specificity of standard PCR reaction; (iii) decrease the number of false positive results during identification of the amplified genetic material such as DNA of tick-transmitted pathogens.

Known from patent description PL218839 B1 is the method for identification of enterohemorrhagic *Escherichia coli* (EHEC) based on the detection of the signal from the probe using a transilluminator that emits UV radiation. From this description there are also known both the sequences of probes as well as sequences of primers allowing for amplification of DNA fragments encoding Shiga toxins ,and their use in identifying EHEC bacteria.

Patent PL220906 B1 discloses a manner of visual diagnostic of microorganisms based on the UV light-dependent detection of signal emitted by probe used in three stage PCR program. The PCR reaction is performed using the 5'-3' exonuclease activity of DNA polymerase and dual labelled probe. The probe is a short oligonucleotide complementary to the amplified sequence and contains at its 5' end the fluorescent reporter FAM excited by UV light, and at the 3' end the fluorescence quencher molecule BHQ1. After the PCR reaction is completed, the fluorescence signal generated by the degraded probe in the tube, is detected using UV transilluminator or other source of UV light at ∼312 nm, as a visible to the eye a greenish-yellow color of the solution in the reaction tube. Importantly, the color change of the solution from colorless to greenish-yellow is a result of the probe degradation and occurs only during PCR reaction which contains DNA complementary to the probe.

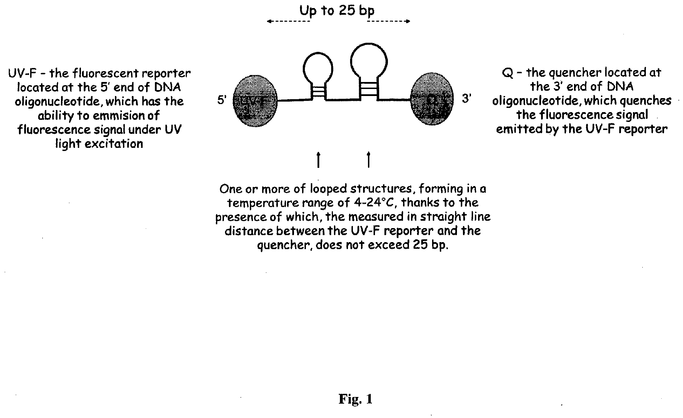

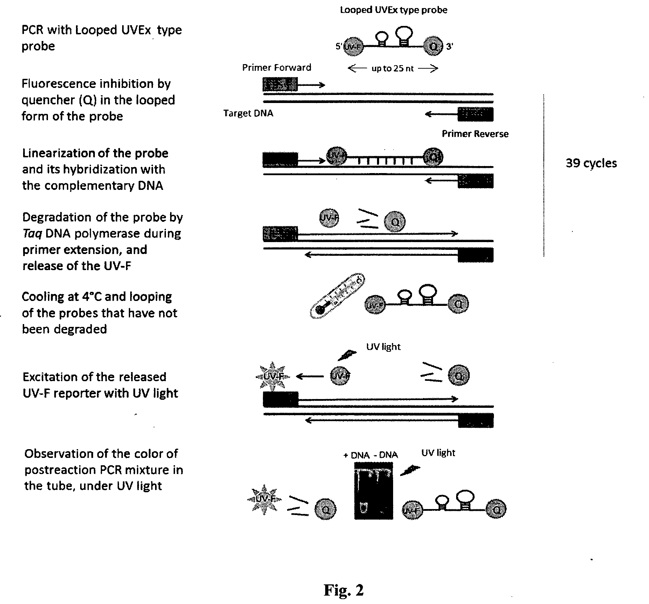

**Figure 1** presents the diagram of the Looped UVEx type probe which is an oligonucleotide of any length, and in the temperature range from 4 to 24°C is able to loop, fold and form at least one looping secondary structure and thus achieve the ΔG (=dG) value* in the range from -1.14 to -8.36 kcal/mole (-1.14 ≥ ΔG(=dG) ≥ -8.36 kcal/mole), thanks to that the measured in straight line distance between excited by UV light fluorescence reporter (UV-F) and its quencher (Q) does not exceed 25 nucleotides (nt). Thanks to such properties, the used in PCR reaction Looped UVEx type probes can be designed as longer oligonucleotides than standard dual labelled linear TaqMan probes (20 - 25nt) and maintain sufficient fluorescence quenching. Such Looped UVEx type probes can be effectively applied in PCR reaction with optimized program expanded to include an additional 4^{th} stage of incubation at 4°C as described in **Table 1.** Importantly, conditions presented in **Table 1** are valid for all indicated here examples of probes that have been applied in the identification of different tick-transmitted pathogens. The application of Looped UVEx type probe in PCR reaction allows for UV light- dependent detection of the amplified PCR product directly in the reaction tube, in accordance with the scheme presented in the **Figure 2****.**

Another specific feature of the Looped UVEx type probe (Fig. 1) is its 5' end fluorescent reporter (UV-F) which instead or in addition to having a fluorescence excitation spectrum in the range of visible light (length 400-700 nm) or/and near infrared (IR) radiation (length 700-1000 nm) has the obligatory ability to emit fluorescence after excitation by ultraviolet light (length below 400 nm). It is also important that quencher (Q) of the Looped UVEx type probe has the possibility to inhibit UV-excited fluorescence emitted by the reporter (UV-F) and does not emit its own fluorescence or emits a fluorescence at the low background level, and the signal of fluorescence is not visible by the eye during the at least four-minute period of UV light exposure of the negative control (sample without the target DNA). The UV-F reporters and quenchers (Q) well-functioning for Looped UVEx type probes are indicated in **Table 2** but not limited to the presented examples. Importantly, the fluorescent signal emitted by the UV light-excited post PCR mixture in the tube, is visible by the eye and its color depends on the selected fluorescent reporter UV-F, as shown in Table 2.

Co-infection or the possibility of simultaneous infection with many pathogens is the main problem related to tick-transmitted microorganisms. Ticks can harbour more than one disease-causing agent and patients can be infected with more than one pathogen at the same time. A research and clinical experience has demonstrated the nearly universal phenomenon of co-infection with multiple tick-borne pathogens in chronic Lyme patients. Despite of *Borrelia* bacteria, these patients may carry different species of genus *Babesia*, *Bartonella*, *Rickettsia* and *Mycoplasma* as well as *Anaplasma phagocytophilum* or *Coxiella burnetii* species. It has been published that as many as 66% of Lyme patients show serologic evidence of co-infection with *Babesia microti.* Diagnosis of Lyme disease at early erythema stage gives a good chance of cure with sufficiently long antibiotic therapy. Co-infection with more than one tick-borne agent results in a more severe clinical presentation and increased organ damage. Such pathogens become more difficult to eradicate. Besides, co-infection prolongs the patient's recovery time and may have immunosuppressive effect such as for example simultaneous infection with *Borrelia* and *Babesia* species. Co-infection may also synergize pathogen-induced lesions in human joints, heart and nerves. There are changes in the clinical presentation of the co-infected patient as compared to when each infection is present individually. Different symptoms and atypical signs may occur resulting in difficulties in treatment and diagnosis. Typical treatment effective in case of single infection, frequently doesn't work in case of co-infections and has to be modified. For example, the antibiotic therapy for Lyme patients does not work effectively in the case of co-infection with *Babesia* and *Bartonella* pathogens. Some even assume, that chronic type of Lyme disease, (especially severe) doesn't exist and all observed symptoms are the consequence of the not diagnosed but present additional infection and wrong treatment. In the face of this problem, a special consideration should be given to the diagnosis of different tick-transmitted pathogens which requires the use of a sensitive and specific diagnostic tool that allow for their accurate identification. The specificity of such a diagnostic test is a big challenge as the tick-transmitted pathogens are closely related. Besides, patient may be infected with other pathogens closely related to these transmitted by ticks. Particular difficulty is the identification of such pathogens based on sequences coding for 16S (18S) rRNA. These sequences are characterized by low variability and their degree of differentiation increase with the increase of the phylogenetic distance between the analyzed microorganisms.

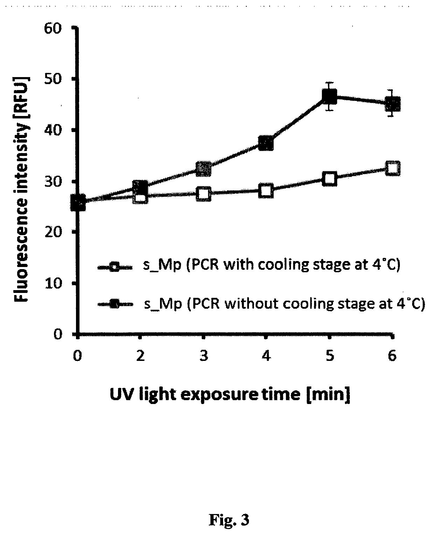

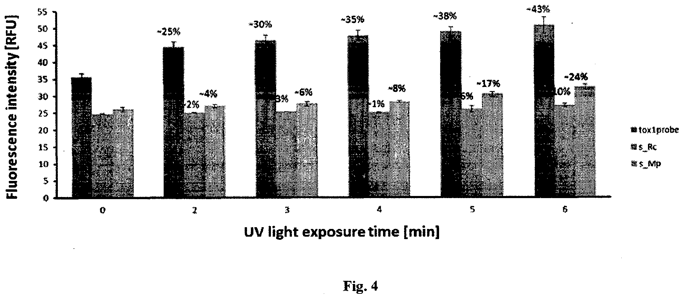

The essence of the disclosure is the Looped UVEx type probe and its application in both the optimized four-stage PCR reaction and in the alternative to standard gel electrophoresis, UV light-dependent detection manner of PCR products (**Fig. 2**). Detection is based on the observation of the UV light-induced color appearance in the tube with postreaction PCR mixture, previously cooled at 4°C. Importantly, such a color appears only in the case of post PCR reaction that include amplified target DNA and depends on attached to the probe UV-F fluorescent reporter. For example AttoRho6G gives yellow-orange color whereas AttoRho14 gives pink (**Table 2**). Both PCR reactions, with and without the target PCR product are easy to detect and distinguish, as in comparison to the color positive sample (+ DNA), the post PCR mixture of minus sample (- DNA) has no color and is transparent under UV light exposure (**Figure 2**). Significant change of the present invention in comparison to patent descriptions no PL218839 and PL220906, is the introduction of the additional obligatory 4^{th} stage in the PCR program that includes minimum three minutes of incubation at 4°C. Importantly, Looped UVEx type probes used in PCR with the additional cooling stage emit lower background fluorescence coming from the probe non-degraded by polymerase (**Fig. 3** and **Fig. 4**). This change has a positive effect on the color readout of the samples, is beneficial for reading results and thus classification of the DNA+ and DNA- samples and time of the UV light exposure which has been extended to four minutes. Essentially, the increase of background fluorescence of Looped UVEx type probes (S_Rc and S_Mp) do not exceed 10% within four minutes of UV light exposure, whereas the background fluorescence of the tox1probe, described in patents PL218839 and PL220906, increases by 25% in the second minute of UV light exposure (**Fig. 4**). Extending the UV light exposure time to four minutes is beneficial for the PCR reaction with a small initial amount of target DNA (in the range of several picogram [pg] and lower) in which the post PCR emitted fluorescence is weak or even absent at the beginning of the UV light exposure and requires longer exposure time to become visible to the eye. Maintaining a low and stable background fluorescence is especially important in the case of negative sample (PCR without target DNA) as produced signal cannot be visible by the eye during whole period of UV light exposure.

An undoubted advantage of the Looped UVEx type probes, in comparison with the applied previously linear TaqMan probes, is their length. Looped UVEx probes can be longer than standard TaqMan probes but importantly able to fold and form secondary structures so that the measured in a straight line distance between the fluorescent reporter and the quencher, in the coiled form of probe, does not exceed 25 nucleotides, in temperature range 4-24°C (**Fig. 1**). Under such conditions, the quencher is in close proximity to the reporter and inhibits its fluorescence effectively as the signal is not visible by the eye during at least 4 minutes of UV light exposure. The inhibition occurs effectively but only in case the probe is not degraded during PCR by *Taq* DNA polymerase.

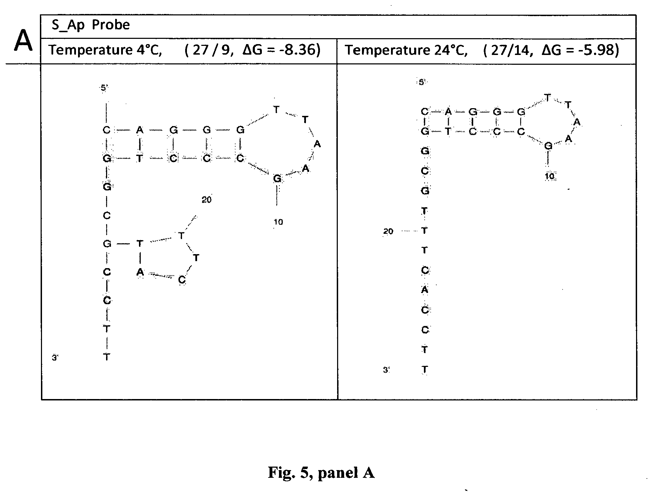

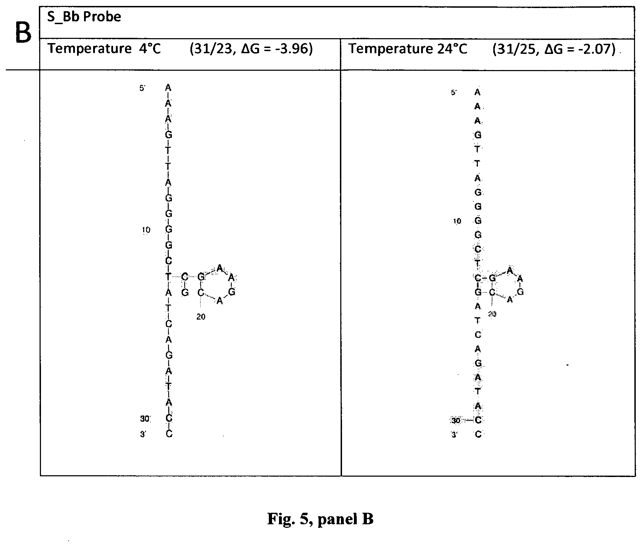

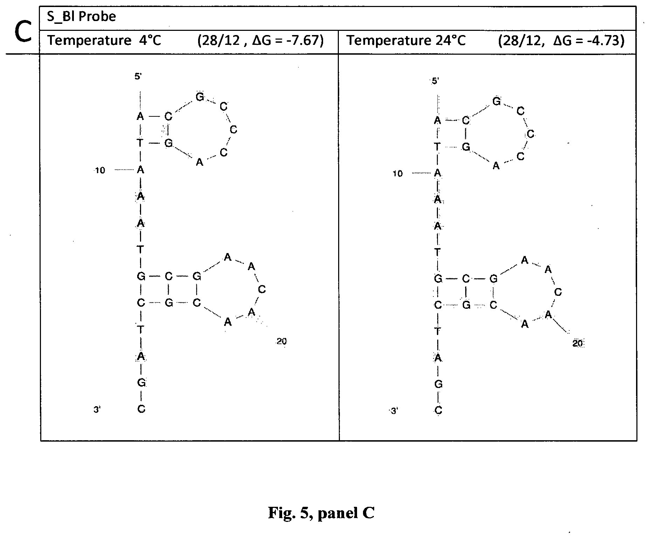

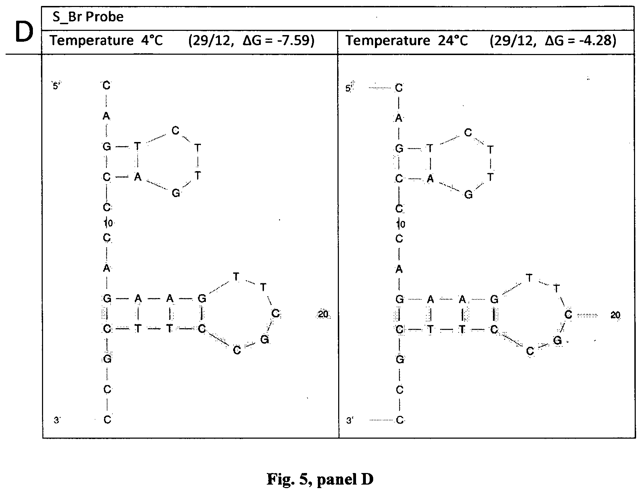

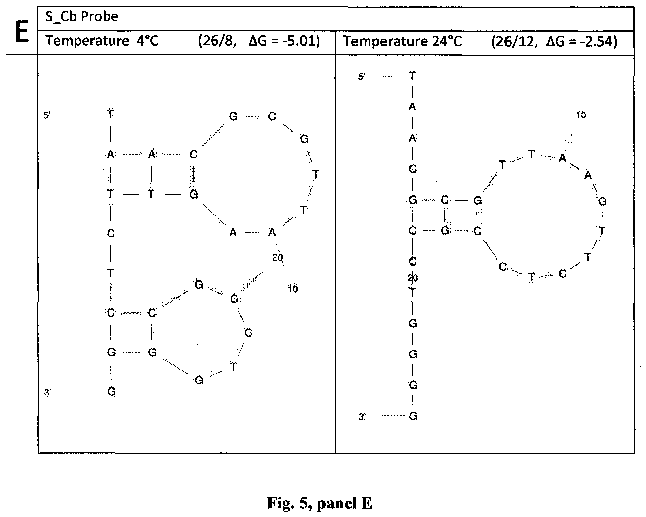

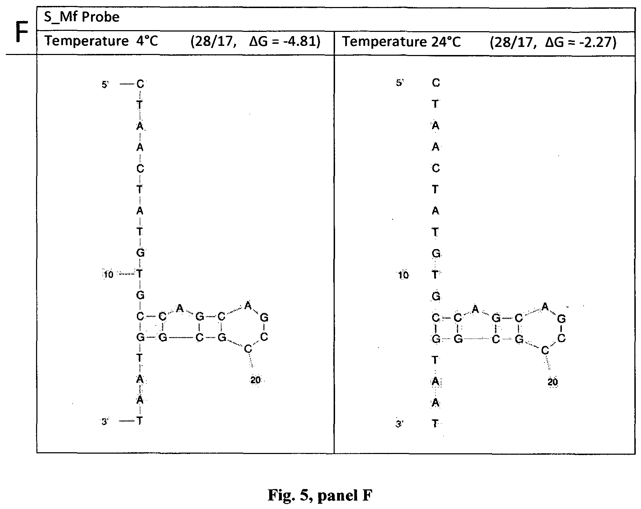

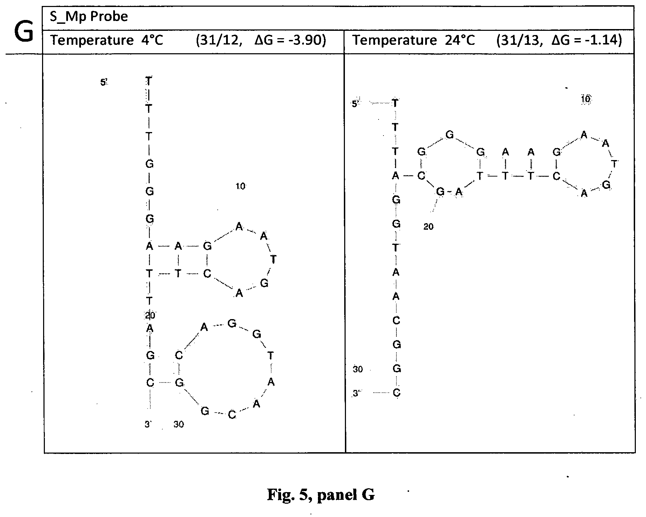

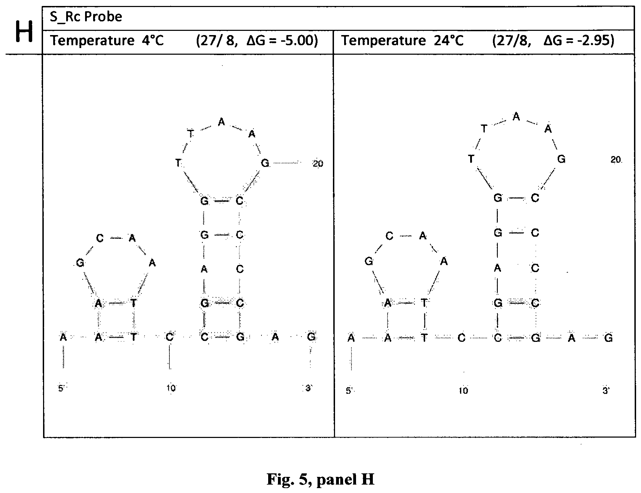

Degradation of the Looped UVEx type probe is effected by 5 'exonuclease activity of the *Taq* DNA polymerase and occurs only if the target DNA is present in the tube and amplified during subsequent cycles of the PCR reaction. The probe is looped at the stage of PCR preparation as everything is performed on ice and linearized during denaturation step of PCR. If the DNA complementary to the probe is in the tube, the probe is hybridized to such a DNA fragment and then degraded by *Taq* DNA polymerase during common annealing and elongation step. As a result of the probe degradation, the molecules of fluorescent reporter and quencher are released and separated so that the quencher is unable to inhibit longer the fluorescence emitted by the UV-F reporter. The amount of the released and separated molecules increases in each PCR cycle and depends on the initial amount of the target DNA in the reaction mixture. If there is no target DNA in the reaction (- DNA) or the initial amount is very small (under the detection level), respectively all or a large number of probe molecules are not degraded during PCR. Importantly, probes that have not been degraded are looped again during the last stage of PCR reaction which is cooling at 4°C (**Table 1**). After the PCR reaction is finished, the chilled post PCR reaction tubes are placed on the source of UV radiation e.g. transilluminator, observed and photographed in the UV light which induces the fluorescence of the UV-F reporters released from the inhibitory effect of the quenchers. The fluorescent signal emitted by reporters is observed in the UV light as an appearance of the coloration of the transparent so far reaction mixture in the tube. No visible coloration of the post PCR reaction mixture means no or too little initial amount of target DNA in the tube. This effect is the result of the last cooling step at 4°C during which the linearized probes undergo a looping and the fluorescence reporter is again in close proximity to quencher (up to 25 nt) and under its inhibitory influence. As a result, despite the excitation of the reporter with UV light, no fluorescence signal is emitted or such a signal is very weak and do not exceed the low level of the background fluorescence, and consequently there is no visible coloration of the reaction mixture. It is important that the Looped UVEx type probes can form secondary structures not only at temperature 4°C but also at room temperature (RT), as UV light exposure is performed at RT and the chilled samples are quickly heated during this analysis (**Fig. 1** **and** **5**). As illustrated in the **Fig. 5**, the looped probes with ΔG values ranging from - 1.14 kcal/mole to -8.36 kcal/mole have been found to have such features and their application in PCR reaction allows to obtain low and stable level of background fluorescence. It is also important that the probes with the ΔG values indicated above are easily linearized during the denaturation phase and hybridized to the target DNA during the simultaneous primers annealing and extension PCR stages (**Table 1**). Considering the increase in background fluorescence of more than 10% after 4 minutes of exposure to UV light, as well as the risk of overheating of the sample and the degradation of DNA and probe during too long exposure, the maximum exposure time of UV light from the transilluminator was estimated and it is 4 minutes. For this reasons, it is very important to read the results and to photograph tested tubes within maximum 4 minutes from the beginning of the UV light exposure. After the PCR reaction is completed and before UV exposure, samples may be stored at 4C for a longer time, e.g. 10, 60 minutes or even overnight. The diagram of the modified PCR reaction with the Looped UvEx type probe is presented in the **Figure 2**.

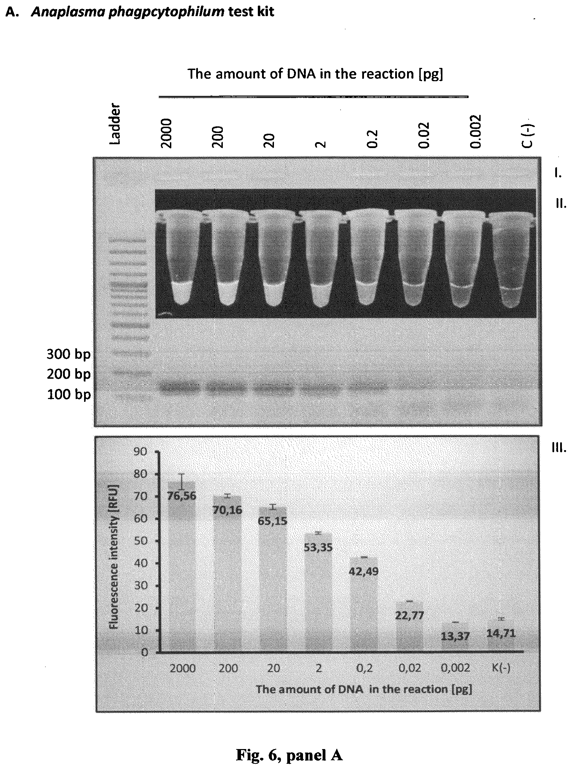

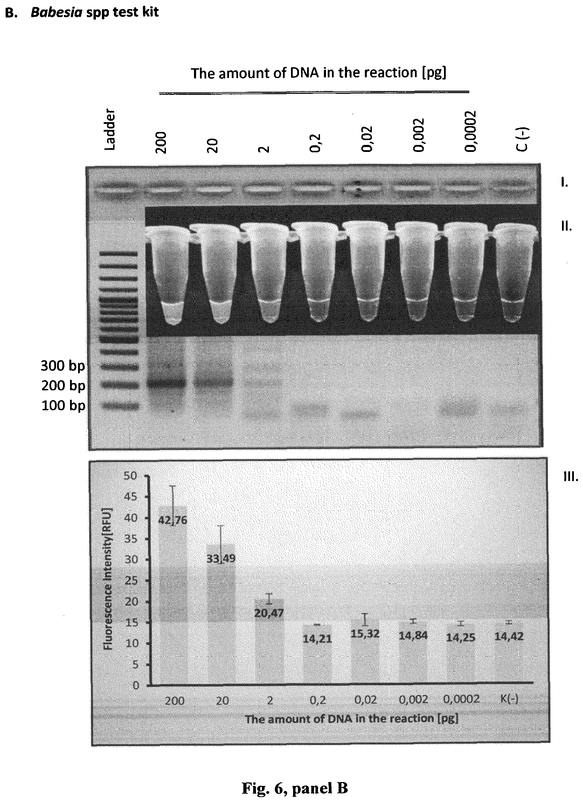

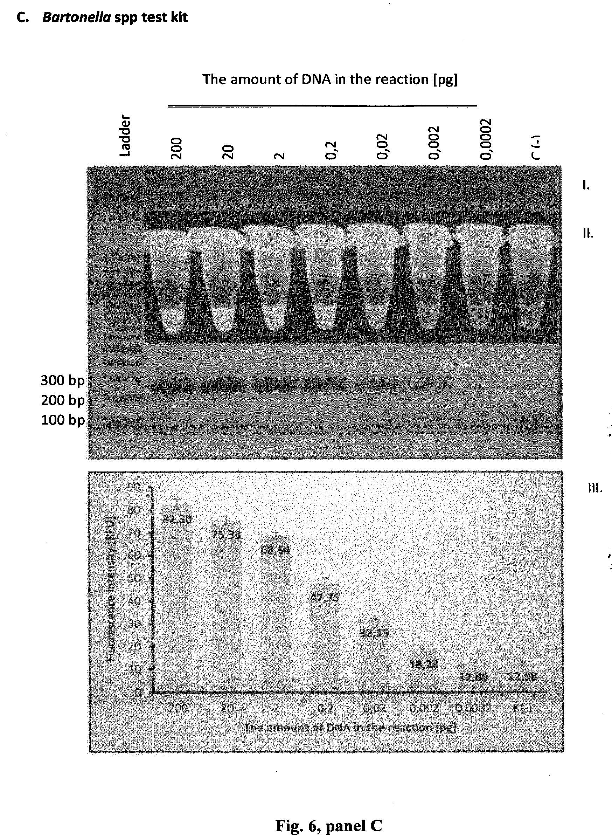

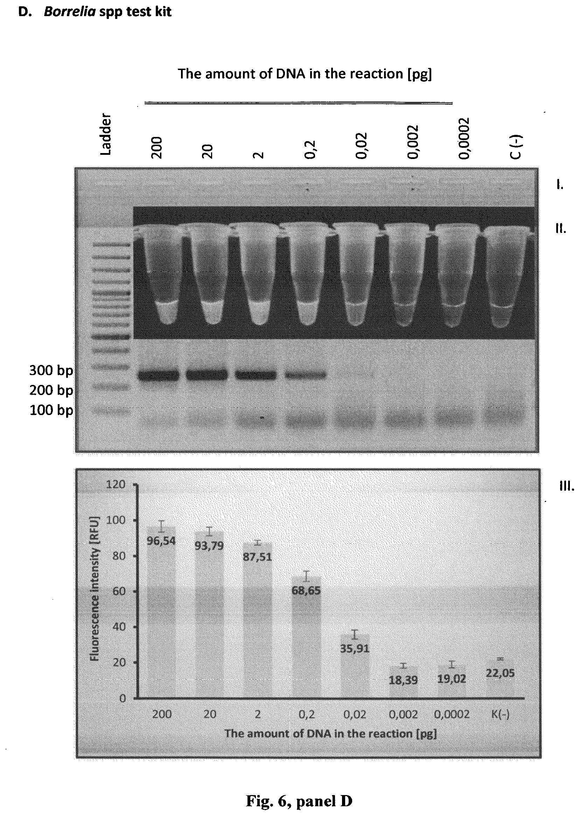

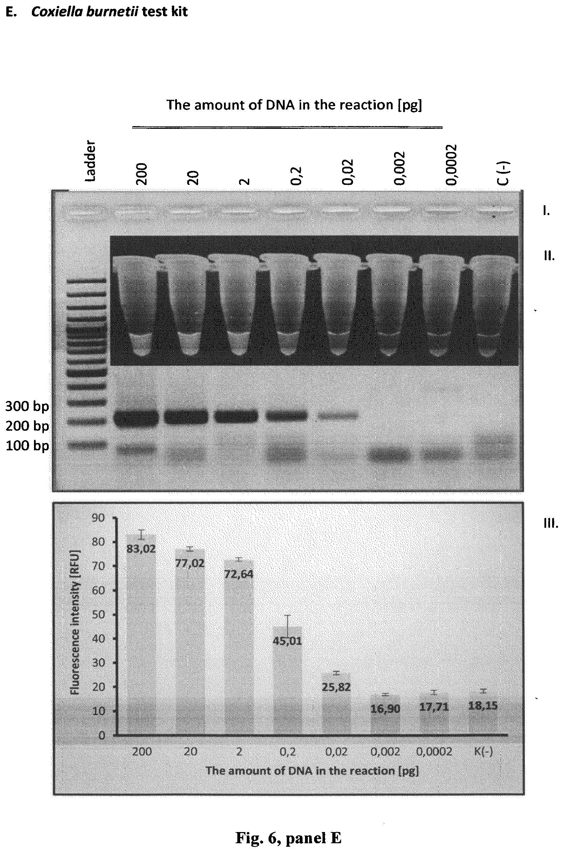

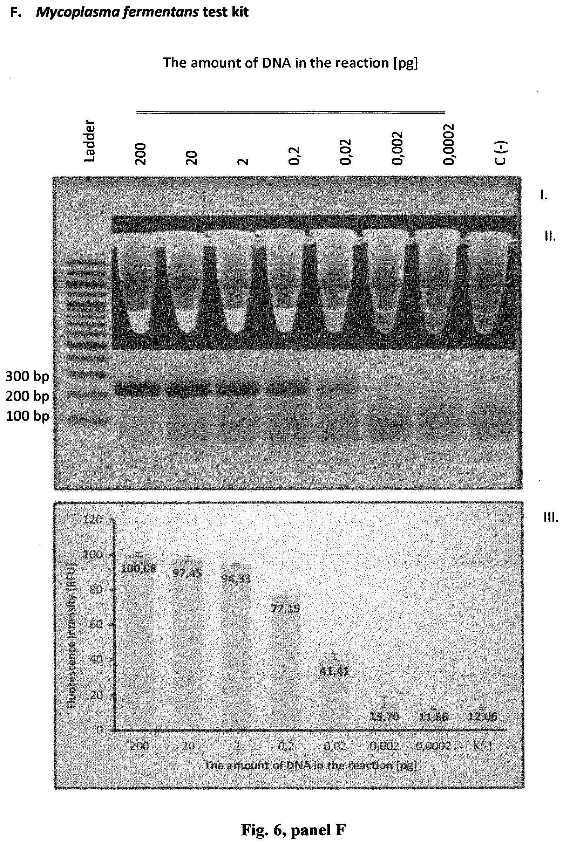

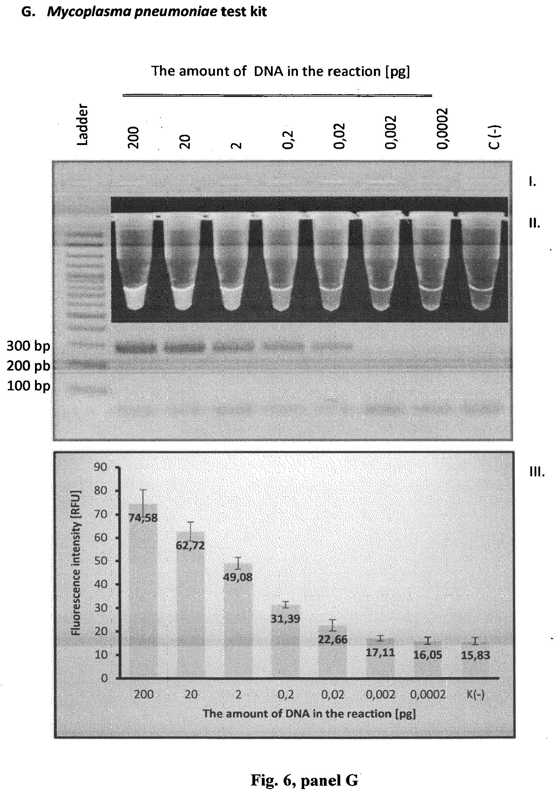

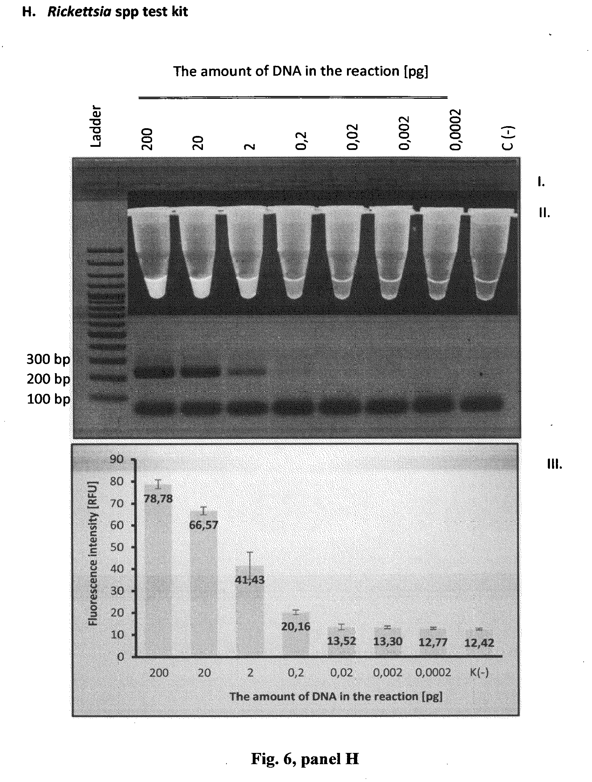

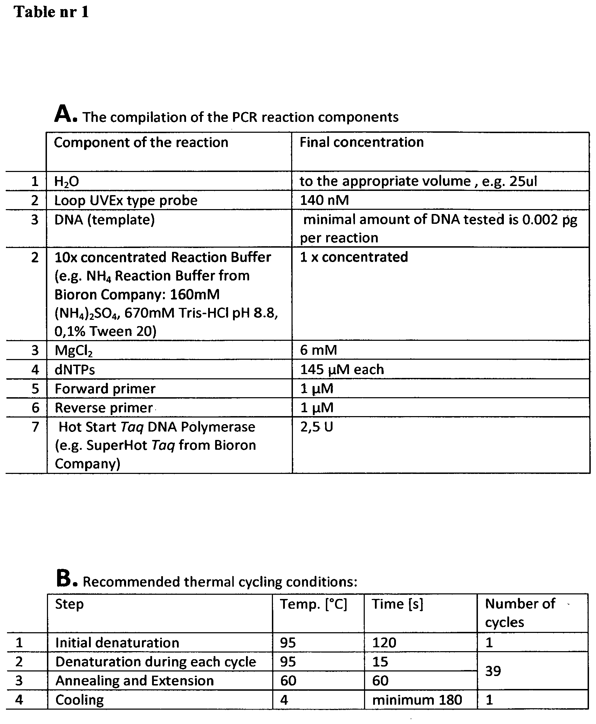

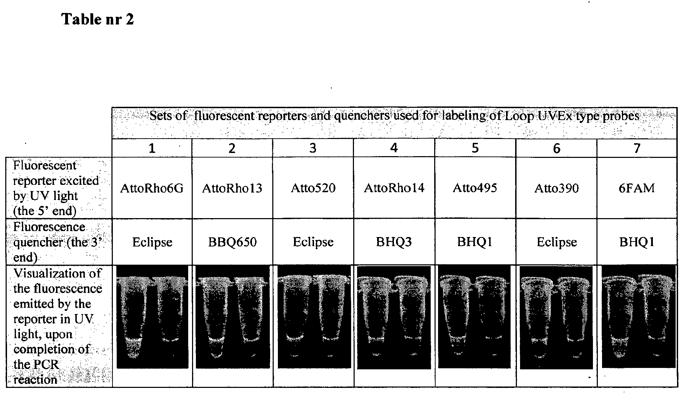

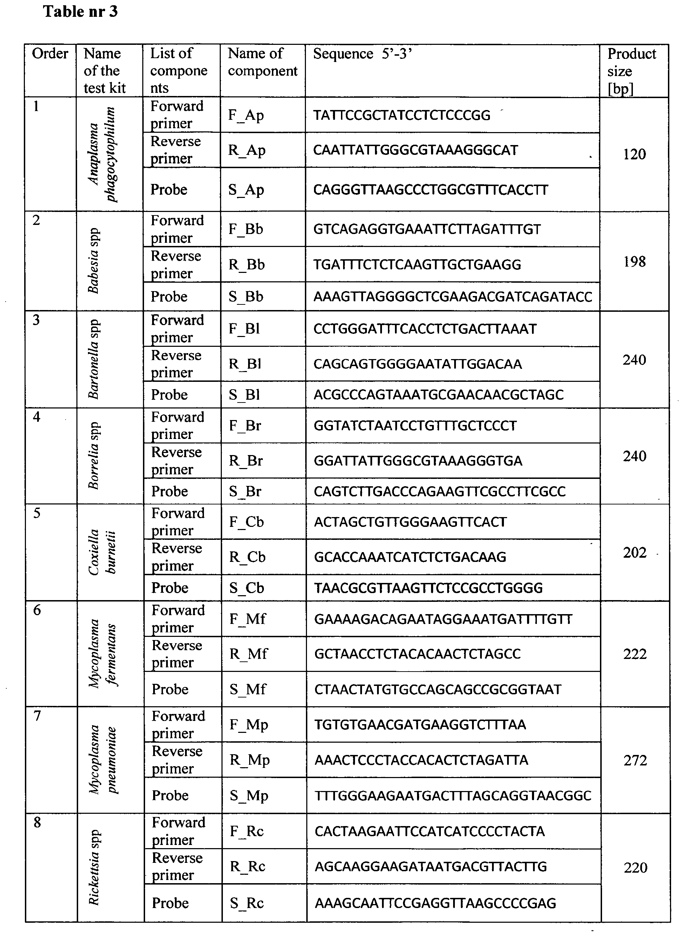

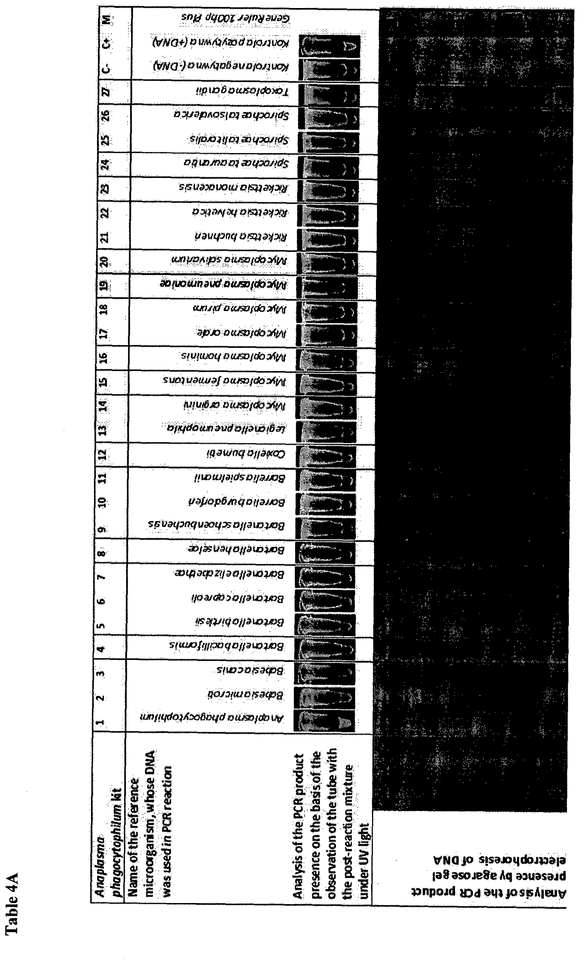

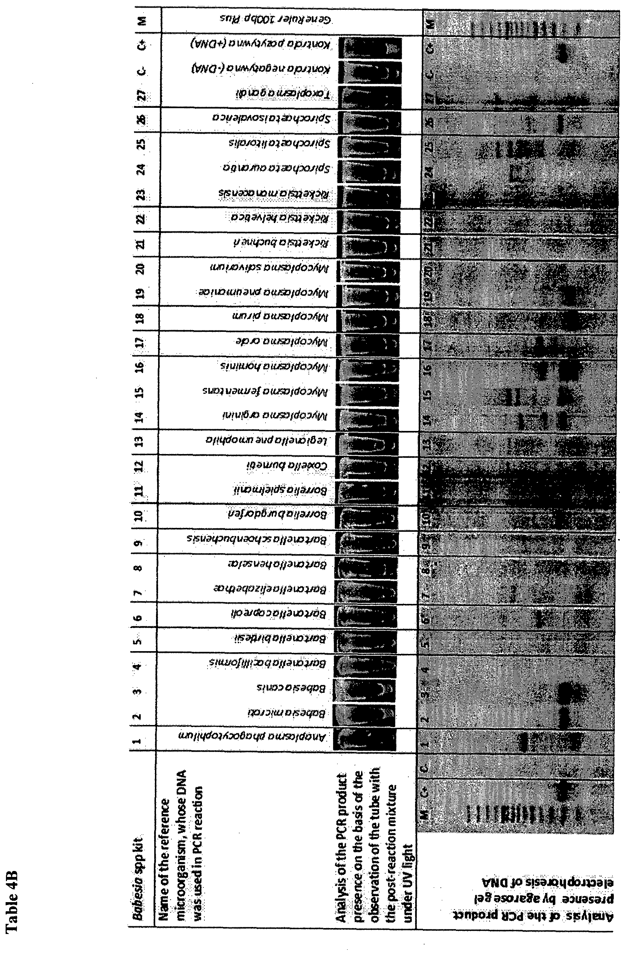

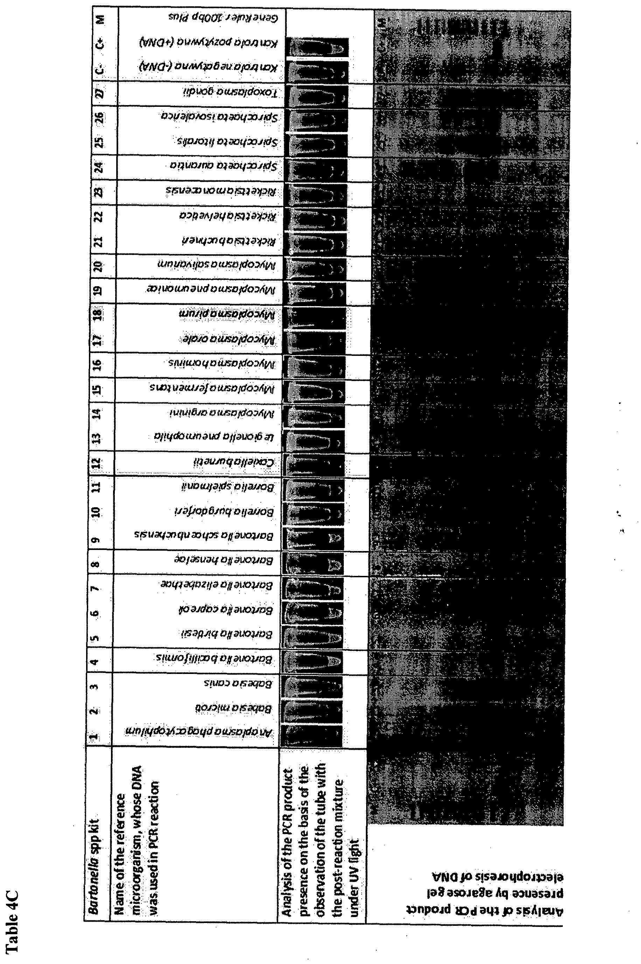

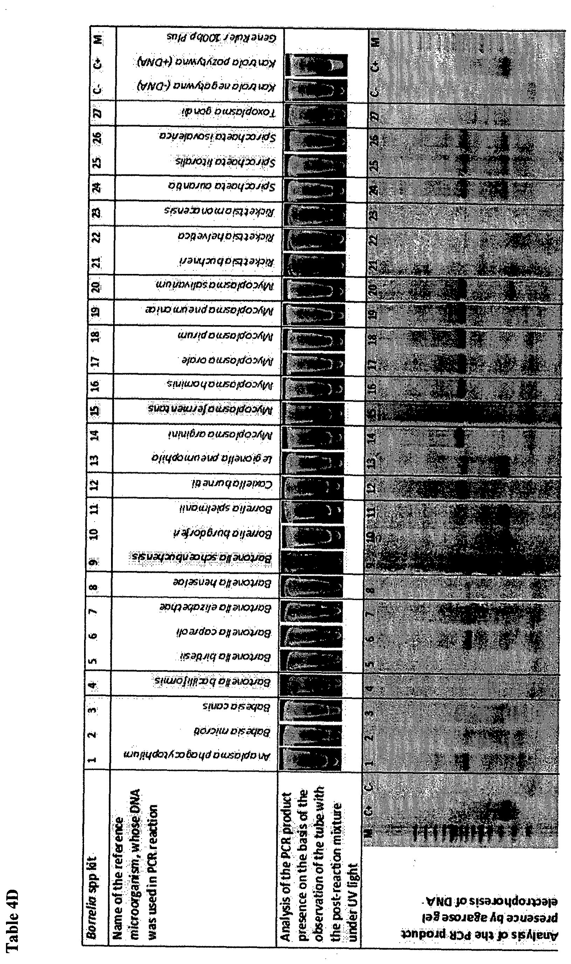

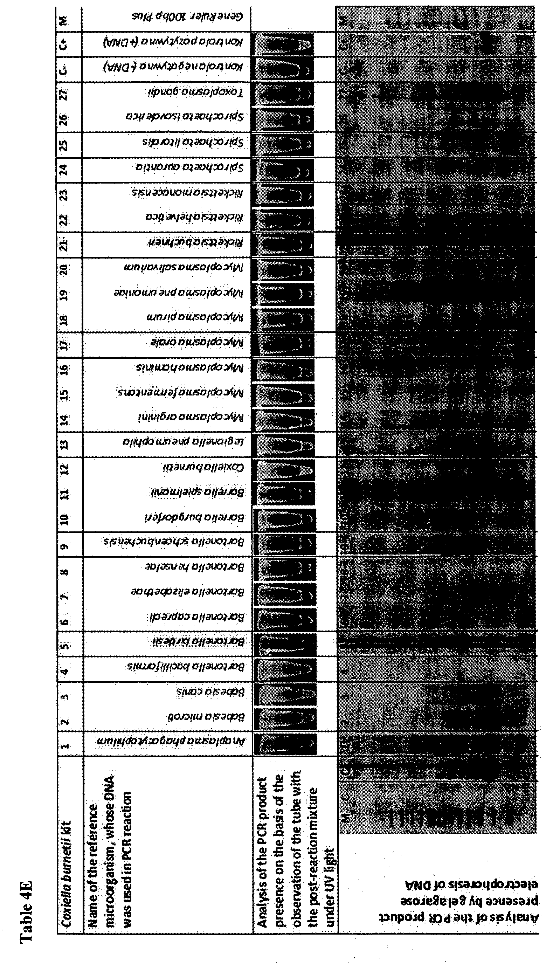

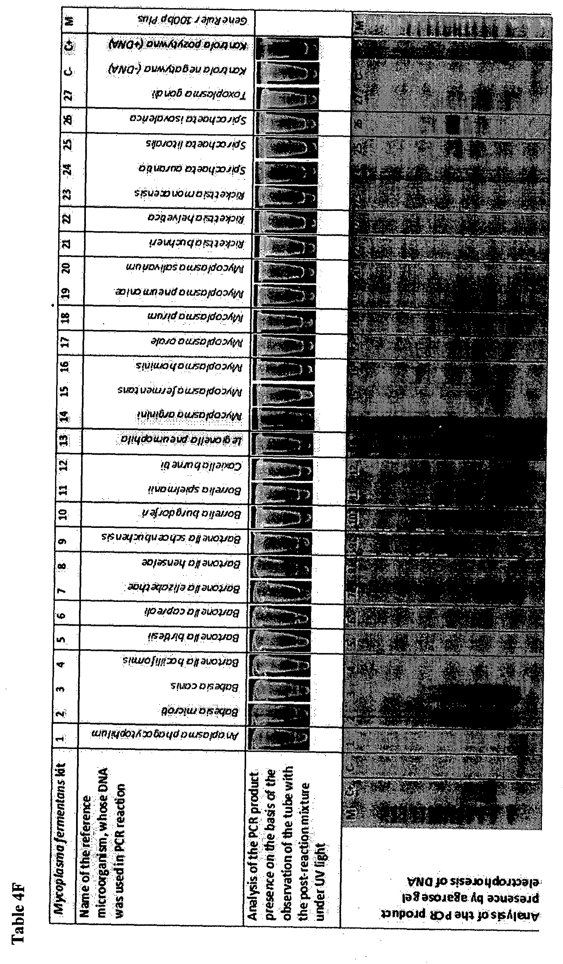

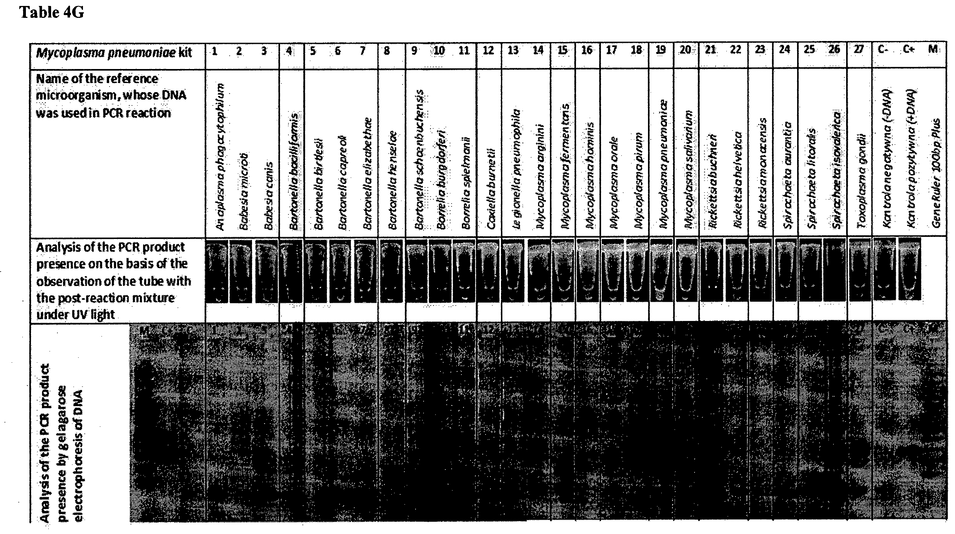

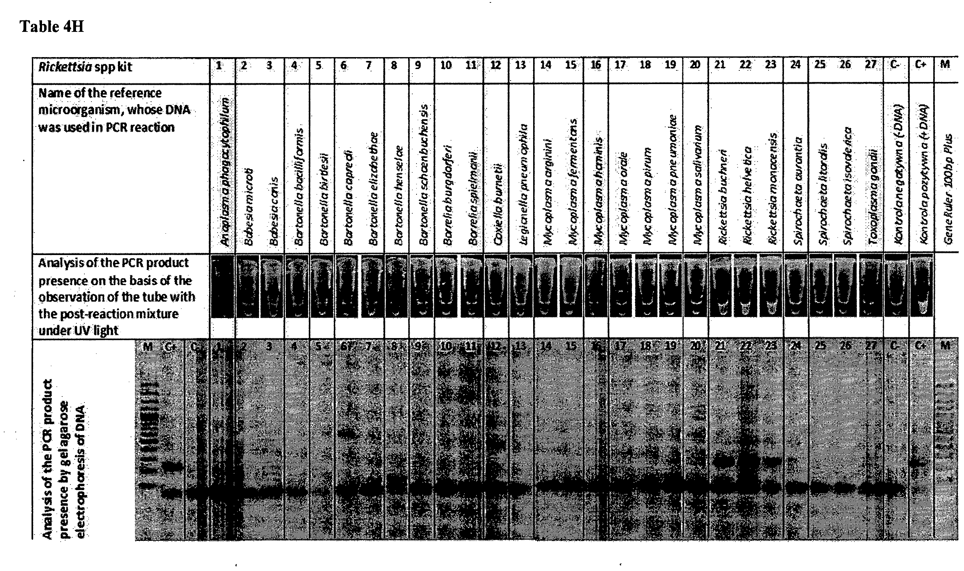

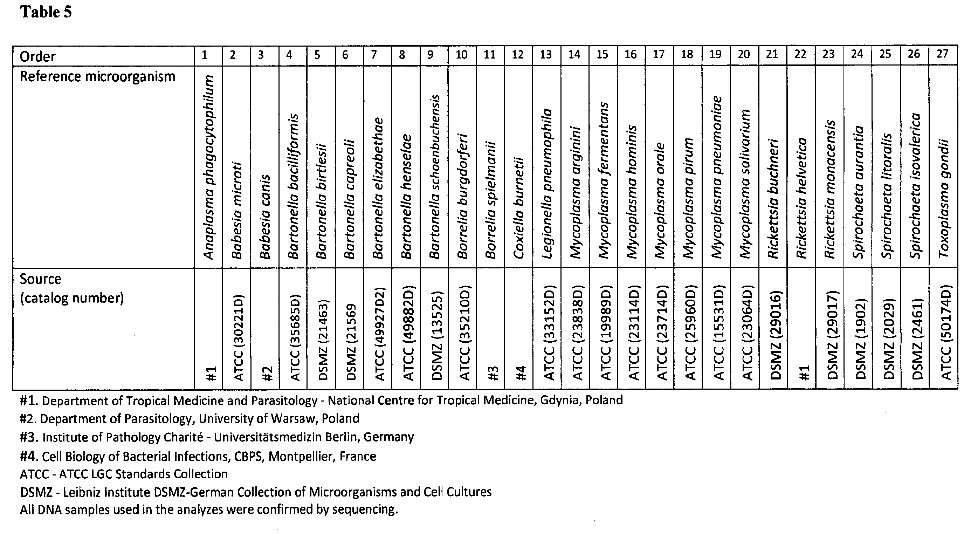

An additional advantage of the Looped UVEx type probe application in the optimized four-step PCR reaction, is possibility to detect PCR product from the one reaction in three different ways. In addition to do standard agarose gel electrophoresis, it is also possible to perform the UV-dependent detection as described above and to read the emitted fluorescence signal using a microplate reader (**Fig. 6**). In the last option, the post PCR mixture is diluted 30 times in nuclease-free water and then the fluorescence emitted by this mixture is measured in a microplate reader using a specific wavelengths of the excitation and emission range. The selection of the appropriate wavelengths depends on the ability of the microplate reader itself but mainly on the used fluorescent reporter and its fluorescence excitation and emission spectra. Importantly, the emitted from the Looped UVEx probe fluorescence signal might be effectively excited by a wavelength of the UV light range but also by the wavelength outside of this range. Each of these three detection methods can by performed separately however the application of the Looped UVEx type probe in PCR reaction is a necessary condition to carry out the fluorescence measurement in microplate reader and to perform analysis based on the observation of the post PCR mixture's color under UV light. The undoubted advantages of the UV light-dependent detection method are: very short time needed for this analysis (up to 4 minutes); no need to purchase expensive high-tech equipment and employ qualified personnel to handle it; and the fact that both the amplification of the PCR product and its detection occur in the same reaction tube without the need to carry the mixture anywhere else. In effect, there is no risk of contaminating the sample after the PCR reaction is complete. But what is important is that all three analyzes can be done concurrently and used as an in-depth supplement to verify the reliability of the results. Such an approach would be extremely useful in diagnosis of pathogens especially those transmitted by ticks, for which both the sensitivity and specificity of the detection method are important and have influence on the final result and thus the diagnosis and recommended treatment.

The object of the invention is the Looped UVEx type probe used for detection of the genetic material that is amplified during PCR reaction with Hot Start *Taq* DNA Polymerase, and may come from the transmitted by ticks pathogens. The Looped UVEx type probe is an oligonucleotide of any length that in the temperature range from 4 to 24°C is able to fold, loop, and form at least one looping secondary structure and thus achieve the ΔG (=dG) value* in the range from -1.14 to -8.36 kcal/mole (-1.14 ≥ ΔG(=dG) ≥ -8.36 kcal/mole). As a result of such a feature the distance between excited by UV light fluorescence reporter (UV-F) and its quencher (Q) does not exceed 25 nucleotides (measured in a straight line) what allows to keep UV-F and Q molecules in close proximity.
A probe which contains at the 5' end the fluorescent reporter UV-F which instead or in addition to having a fluorescence excitation spectrum in the range of visible light (length 400-700 nm) or/and near infrared (IR) radiation (length 700-1000 nm) has the obligatory ability to emit fluorescence after excitation by ultraviolet light (length below 400 nm). At the 3' end the probe contains quencher (Q) that has the possibility to inhibit UV-excited fluorescence emitted by the reporter (UV-F) and does not emit its own fluorescence or emits a fluorescence at the low background level, and the signal of this fluorescence is not visible by the eye during the at least four-minute period of UV light exposure of the tube with negative control (sample without the target DNA).

A probe that in the temperature range from 4 to 24°C achieves the ΔG (=dG) value* in the range from -1.14 to -8.36 kcal/mole (-1.14 ≥ ΔG(=dG) ≥ -8.36 kcal/mole).

A probe characterized in that its fluorescent reporter UV-F might be selected from the following reporters: AttoRho6G, AttoRho13, Atto520, AttoRho14, Atto495, Atto390, 6FAM.

A probe characterized in that its quencher might be selected from the following molecules: Eclipse, BBQ650, BHQ3.

A probe might be selected from the sequences presented in Table 3.

Preferably, a probe characterized in that its melting temperature generated by the Primer3 program is maintained at, but not limited to, the level 70 ± 3°C.

A probe which in the PCR reaction carried out according to the guidelines of Table 1 and in the presence of the complementary target DNA, is degraded by the *Taq* DNA polymerase and emit a fluorescence signal visible as a coloring of the reaction mixture during four minutes exposure to UV light.

A pair of forward and reverse primers may be selected from the sequences shown in Table 3.

A pair of primers characterized in that the melting temperature of each of the primer (forward and reverse) is maintained at the level 60 ± 3°C.

A pair of primers which in the PCR reaction carried out according to the guidelines of Table 1 and in the presence of the complementary target DNA allows amplification of the DNA fragment bounded by these primers.

A method for identification of tick transmitted pathogens characterized in that, the Looped UVEx probe that is coiled and forms secondary structures during preparation of the PCR reaction on ice, undergoes linearization during initial and cyclic DNA denaturation at 95°C, followed by hybridization with the complementary DNA and further degradation during the stage of simultaneous primers annealing and extension that is conducted for 60 sec at 60°C. During this stage the bound to the target DNA probe is degraded by Hot Start *Taq* DNA Polymerase. In effect, the molecules of fluorescent reporter and quencher are released and separated so that the quencher is unable to inhibit longer the fluorescence emitted by the UV-F reporter. The amount of the released and separated molecules increases in each of the target thirty-nine PCR cycles and depends on the initial amount of the target DNA in the reaction mixture. Probes that have not been degraded due to the lack of the target DNA in the reaction or its small initial amount (below detection level) are looped again and form secondary structures during the last PCR cooling stage at 4°C which is conducted for at least 3 minutes (Table 1). After the PCR reaction is finished, the chilled post PCR reaction tubes are placed on the source of UV radiation (< 400 nm) e.g. transilluminator, observed under UV light for maximum 4 minutes and photographed.

During exposure, UV light induces the fluorescence of the UV-F reporters released from the inhibitory effect of the quenchers. The fluorescent signal emitted by reporters is observed in the UV light as an appearance of the coloration of the transparent up to now reaction mixture in the tube. Importantly the color of the mixture could be different and depends on the used fluorescent reporter UV-F. Lack of the visible coloration of the post PCR reaction mixture means no or too little initial amount of target DNA in the test tube. This effect is the result of the last cooling step at 4°C during which the linearized probes undergo a looping and the fluorescence reporter is again in close proximity to quencher (up to 25 nt) and under its inhibitory influence. In effect, no fluorescence signal is emitted after UV light excitation or such a signal is very weak and do not exceed the low level of the background fluorescence, and consequently there is no visible by the eye coloration of the reaction mixture.

A method characterized in that it is based on the PCR reaction with *Taq* DNA polymerase, Looped UVEx type probe and a pair of primers defined above.

The use of described above Looped UVEx type probes for the detection and/or diagnostics of tick-transmitted pathogens are presented in Table 5.

The use of described above primers for the detection and/or diagnostics of tick-transmitted pathogens.

### Brief description of the drawings and tables:

**Fig. 1** - presents the schema of the Looped UVEx type probe in a folded, looped and non-degraded form.
**Fig. 2** - presents the schema of PCR reaction with the Looped UVEx type probe and the process of detection of the PCR product under UV light, after cooling the post-reaction mixture at 4°C. The Looped UVEx type probe used in the reaction is a DNA probe of any length, however has the ability to form a minimum of one looped structure in the temperature range of 4-24°C, which presence results in reduction of the distance, up to a maximum of 25 nucleotides, between UV light-excited fluorescent reporter (UV-F at the 5' end) and the appropriately selected fluorescence quencher (Q at the 3' end). The formation of looped structure allows the quencher to be in close proximity to fluorescent reporter and to inhibit the emission of fluorescent signal. This happens in the absence of the target DNA in the reaction (-DNA) when no degradation of the probe by the polymerase is observed. The fluorescence signal is visible to "the naked eye", as a change in color of the post-reaction mixture in a UV light-exposed tube, only if the target DNA is present in the sample and its amplification is effective (+DNA).
**Fig. 3** - presents a comparison of the levels of reaction background fluorescence that were emitted by Looped UVEx type probe (S_Mp) in the negative control sample (-DNA), after 3 minutes of cooling in the temperature 4°C (empty squares) or without the cooling step (filled squares), and following UV light exposure. Fluorescence measurements were made at wavelength 495 nm / 520 nm using a plate reader.
Introduction of the cooling step results in the reduction of the level of background fluorescence.
**Fig. 4** - presents a comparison of background fluorescence levels emitted by the probe tox1probe known from the patents PL218839 B1 and PL220906 B1 and two Looped UVEx type probes S_Rc and S_Mp of the present application. Fluorescence measurements were performed in cooled post PCR reaction mixtures of negative controls (- DNA), exposed to UV light for the indicted lengths of time, and made at wavelength 495 nm / 520 nm using a plate reader. The tox1probe does not belong to the Looped UVEx type probes as its ΔG values calculated by Mfold software are -1.55 and 0.98 kcal/mole at 4°C and 24 °C, respectively. As can be seen from the graph, the Looped UVEx type probes present both lower initial background fluorescence (time 0) and lower fluorescence growth over time of UV exposure. Taking into account the level of background fluorescence, Looped UVEx type probes are more preferable than probe tox1probe, with respect to UV light-dependent detection method.
**Fig. 5** - (panels A-F) presents examples of Looped UVEx type probes in their folded, coiled forms at both temperatures 4 and 24°C. The ΔG values of these probes have been calculated by Mfold software and both are in the range -1.14 ≥ ΔG(=dG) ≥ -8.36 kcal/mole. The number of nucleotides separating the reporter from the quencher in both linear and coiled forms is given in parentheses.
**Fig. 6** - presents the results of the analysis of the specificity of the indicated sets of Looped UVEx type probes and primers, designed for amplification of the DNA isolated from different tick-transmitted pathogens (panels A-H). Detection of the amplified PCR products was made by: (I) agarose gel electrophoresis, (II) observation of UV light-induced color change of the post PCR reaction mixture from transparent to orange-yellow (III) fluorescence measurements of the diluted 30 times post PCR reaction mixtures, at wavelength 534 nm / 559 nm, using a plate reader. In this experiment, probes were labeled with fluorescent reporter AttoRho6G, at 5' end, and with quencher Eclipse, at 3' end.
**Table 1** - presents the PCR reaction conditions optimized for Looped UVEx type probes. PCR reaction components are presented in part A, and program conditions in part B.
**Table 2** - presents the sets of commercially available fluorescent reporters (UV-F) and quenchers (Q) used for labeling of Looped UVEx type probes. The presented combinations of reporters and quenchers were tested on a set of two primers and probe, designed to detect *Borrelia* spp., under conditions described in Table 3. The photos were taken after 4 minutes of exposure to UV light from the transilluminator (302 nm). Contrary to DNA-free control samples (- DNA), the test samples contained DNA isolated from bacteria *Borrelia burgdorferi.* The genetic material has been obtained from ATCC collection (no 35210D).
**Table 3** - presents the eight examples of test kits of Looped UVEx type probes and primers, used in the optimized PCR reaction for amplification and detection of DNA isolated from tick-borne pathogens.
**Table 4** - presents results of:
   A - specificity analysis of the test set called *Anaplasma phagocytophilum*
   B - specificity analysis of the test set called *Babesia* spp
   C - specificity analysis of the test set called *Bartonella* spp
   D - specificity analysis of the test set called *Borrelia* spp
   E - specificity analysis of the test set called *Coxiella burnetii*
   F - specificity analysis of the test set called *Mycoplasma fermentans*
   G - specificity analysis of the test set called *Mycoplasma pneumoniae*
   H - specificity analysis of the test set called *Rickettsia* spp
**Table 5** - presents a list of reference strains whose DNA was used for testing the test kits presented in Table 3.

The following exemplary embodiments are illustrative only, hence are not intended to limit the scope of the present invention.

Embodiments of the application of Looped UVEx type probes, characterize by the ΔG value* in the range from -1.14 to -8.36 kcal/mole, in the temperature range from 4 to 24°C (**Fig. 1**), in the optimized PCR reaction are illustrated in **Fig. 6** and in **Tables 3 and 4.** These are examples of using the Looped UVEx type probes for identification of genetic material coming from pathogens transmitted by ticks. The experiments were conducted with eight newly designed sets of probes and primers, aimed at detecting and amplifying the DNA of the following pathogens: 1. *Anaplasma phagocytuphilum*, 2. *Babesia* spp, 3. *Bartonella* spp, 4. *Borrelia* spp, 5. *Coxiella burnetii*, 6. *Mycoplasma fermentans*, 7. *Mycoplasma pneumoniae* oraz 8. *Rickettsia* spp. In all these cases, the probes and primers were designed to the gene sequences coding for rRNAs 16S or 18S in the case of *Babesia* and their sequences are presented in **Table 3.** All test kits that contain the word 'spp' whitin the name can be used to detect more than one species of pathogens within the genus group. All the presented sets of probes and primers have been applied in the PCR reaction optimized according to the conditions set out in the **Table 1**, and tested on reference DNA samples obtained from different scientific centers (**Table 5**). Importantly, the generated by the Primer3 program melting temperatures are maintained at the level 70 ± 3°C for all presented probes and at the level 60 ± 3°C for all presented primers. Thanks to this, it was possible to establish universal PCR reaction conditions for all developed test kits. In contrast to the classical PCR technique, the optimized PCR reaction with Looped UVEx type probes is characterized by: simultaneous execution of the primers annealing and elongation steps (for the same length of time and at the same temperature = 60°C); increasing the number of cycles from the standard 36 to 39 to improve sensitivity; and introducing the obligatory cooling step by at least 3 minutes at 4°C to allow the non-degrading probes to loop again and form secondary structures (**Table 1**). Due to the absence of a typical distinct elongation step at the optimum temperature for the polymerase, and due to an increase in the number of cycles, it is important to analyze the range of temperatures in which the selected polymerases operate and it is recommended to increase the standard amount of polymerase units to a minimum of 2.5U. So far two different Hot Start *Taq* DNA polymerases (SuperHot *Taq* by Bioron GmbH and *TaqNovaHS* by Blirt S.A.) have been successfully tested in the above-described conditions, although other polymerases with similar properties may also be used.

The sensitivity analysis of the optimized PCR technique with the sets of probes and primers presented in Table 3, showed that application of Looped UVEx type probe had a positive effect on the sensitivity of the PCR method and allowed detecting small initial DNA amounts such as 0.02 pg (Fig 6). This was possible thanks to some reasons: the reduction of background fluorescence, prolongation of UV exposure time, change in the number of cycles from 28 described previously (patents no PL218839 and PL220906) to 39, and the ability to perform triple detection of the PCR products. Importantly, application of high number of cycles (above standard 36) in classical PCR reaction is not recommended due to the increased risk of occurrence of non-specific PCR products that disturb and often even prevent correct interpretation of results using agarose gel electrophoresis. An example of such a situation is the results obtained for the *Babesia* spp set of probe and primers presented in **Fig. 6** **on panel B.** Due to the presence of too many non-specific PCR products after finishing the PCR reaction with an initial DNA amount of 2 pg, it can not be clearly established whether the target PCR product was actually amplified, solely on the basis of images from agarose gel electrophoresis analysis (**Fig. 6****, panel B**). These limitations do not apply to the PCR reaction with the Looped UVEx type probe, since in this case the correct reading of the results is possible by using two different probe-dependent detection methods. As a result of both the UV-dependent analysis and observation of the color change of the post PCR reaction mixture from colorless to orange-yellow, as well as the fluorescence measurements at a wavelength of 559 nm using a plate reader, it was possible to interpret properly the results and clearly confirm the presence of the target product (**Fig. 6****, panel B**). The advantage of the first probe-dependent detection method depends on: the shorter time it takes to complete the test and obtain final results; the absence of the need to carry the mixture from the test tube, and thus the minimization of the risk of sample contamination, and finally the lack of the need to use an expensive instrument such as a microplate reader or to employ qualified personnel for its service.

The specificity of PCR reaction and consequently the number of the amplified different products depend on the quality and degradation level of the used DNA, and the presence in the analyzed sample, of the genetic material highly similar to target DNA, but e.g coming from other closely related organisms. It has been shown that in the case of tick-borne pathogens, the non-specificity of the developed PCR tests is a big problem. In several cases, detecting the presence of DNA from the *Anaplasma phagocytophilum* bacterium, false positive results were obtained in samples containing the DNA of the other tick-transmitted bacteria such as *Rickettsia rickettsii* or *Bartonella henselae*. Besides, the non-specific PCR products may also appear due to the presence in the sample of DNA coming from closely related organisms but not necessarily transmitted by ticks. In the classical PCR reaction, when PCR products are detected by agarose gel electrophoresis, there is no possibility to distinguish between specific and non-specific PCR products if they are similar in size. The solution to this problem is the application of the Looped UVEx type probe in the optimized PCR reaction. Analysis of the specificity of the developed sets of probes and primers have been performed on the reference material isolated from the microorganisms listed in the Table 5. This showed that the use of the Looped UVEx type probe in the optimized PCR reaction and the following UV light-dependent detection of PCR products, improve the specificity of the tests, facilitate the reading of results and reduces the risk of misinterpretation and occurrence of false-positive results. Contrary to the difficulty of interpreting the results from the images of agarose gels, the UV-dependent detection of PCR products allows for unambiguous confirmation of the presence or absence of a specific product as indicated in **Table 4.** During test of the set of probe and primers designed for detection of DNA from *Anaplasma phagocytophilum* (Table 4A), the agarose gel electrophoresis results suggested the presence of the target product in the sample 18, so in the presence of DNA isolated from *Mycoplasma pirum*. In truth, the UV light-dependent detection method has not confirmed this result. Analogous false-positive results were obtained with agarose gel electrophoresis in the case of tests: *Babesia* spp - samples 8 and 19 (**Table 4B**), *Borrelia* spp. - samples 13, 24, 25, 26 (**Table 4D**) and *Mycoplasma pneumoniae* samples 8 and 18 (**Table 4H**). Importantly, the analysis of post PCR reaction mixtures in the UV light allowed to exclude all false positive results. These results demonstrate that the use of a Looped UVEx type probe in the optimized PCR reaction, significantly improves the specificity of this technique and reduces the risk of misinterpretation even in the presence of a non-specific product of a similar size to the correct product (**Table 4H, samples 18 and 19**). Undoubtedly, the use of the looped UVEx type probes in PCR assays can provide many benefits in the field of molecular diagnostics of pathogenic microorganisms. Furthermore, the benefits of using this type of probes in PCR-based assays can occur wherever non-specific PCR products occur.

### References

Ram S, Singh RL, Shanker R. In silico comparison of real-time PCR probes for detection of pathogens In Silico Biol. 2008;8(3-4):251-9.
Nejman-Faleńczyk B, Bloch S, Januszkiewicz A, W 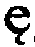 grzyn A, W 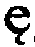 grzyn G. A simple and rapid procedure for the detection of genes encoding Shiga toxins and other specific DNA sequences. Toxins (Basel). 2015 Nov 13;7(11):4745-57
Jiang Y, Ke C, Mieczkowski PA, Marszalek PE. Detecting ultraviolet damage in single DNA molecules by atomic force microscopy. Biophys J. 2007 Sep 1;93(5):1758-67. Ep 2007 May 4.
Burrascano JJ Jr. Diagnostic Hints And Treatment Guidelines For Lyme And Other Tick Borne Illnesses,2005 MANAGING LYME DISEASE, 15th edition
Gray JS, Dautel H, Estrada-Pena A, Kahl O, Lindgren E. Effects of Climate Change on Ticks and Tick-Borne Diseases in Europe. In: Interdisciplinary Perspectives on Infectious Diseases Vol. 2009, Article ID 593232
Nicolson GL, Diagnosis and Therapy of Chronic Systemic Co-infections in Lyme Disease and Other Tick-Borne Infectious Diseases, Townsend Letter 2007 April; 93-98
Clarridge JE. Impact of 16 S rRNA Gene Sequence Analysis for Identification of Bacteria, Clinical Microbiology and Infectious Diseases, vol. 2004 17(4)
Fazekas AJ, Steeves R, Newmaster SG. Improving sequencing quality from PCR products containing long mononucleotide repeats. BioTechniques 2010, 48, 277-285.
Lorenz TC. Polymerase Chain Reaction: Basic Protocol Plus Troubleshooting and Optimization Strategies. J. Vis. Exp. 2012, 63, e3998.
M. Zuker. Mfold web server for nucleic acid folding and hybridization prediction. Nucleic Acids Res. 31 (13), 3406-3415, 2003.
Massung RF, Slater KG. Comparison of PCR assays for detection of the agent of human granulocytic ehrlichiosis, Anaplasma. phagocytophilum. J. Clin. Microbiol. 2003, 41, 717-722.
Qurollo BA, Riggins D, Comyn A, Zewde MT, Breitschwerdt EB. Development and validation of a sensitive and specific sodB-based quantitative PCR assay for molecular detection of Ehrlichia. species. J. Clin. Microbiol. 2014, 52, 4030-4032.

## Claims

1. Looped UVEx type probe (s) for detecting tick-borne pathogen genetic material, that is amplified during PCR reaction by "Hot Start" type *Taq* DNA polymerase, the (each) probe **characterized by** being an oligonucleotide of any length which folds at temperature range 4-24°C and forms secondary structures (including looping) assuming the limit values ΔG (dG) from the range -1.14 ≥ ΔG ≥ -8.36 kcal/mole, whereby the distance, in straight line, between the fluorescent reporter (UV-F) and the fluorescence quencher (Q), in a coiled probe, does not exceed 25 nucleotides (nt), while the probe (s) comprise(s) following nucleotides:
5' CAGGGTTAAGCCCTGGCGTTTCACCTT 3' named S_Ap and/or
5' AAAGTTAGGGGCTCGAAGACGATCAGATACC 3' named S_Bb and/or,
5' ACGCCCAGTAAATGCGAACAACGCTAGC 3' named S_Bl and/or,,
5' CAGTCTTGACCCAGAAGTTCGCCTTCGCC 3' named S_Br and/or,
5' TAACGCGTTAAGTTCTCCGCCTGGGG 3' named S_Cb and/or,
5' CTAACTATGTGCCAGCAGCCGCGGTAAT 3' named S_Mf and/or,
5' TTTGGGAAGAATGACTTTAGCAGGTAACGGC 3' named S_Mp and/or
5' AAAGCAATTCCGAGGTTAAGCCCCGAG 3' named S_Rc.

2. Probe according to the claim 1, **characterized by** the limit value of ΔG for the probe will be greater than or equal to -8.36 kcal / mol and will be lesser than or equal to - 1.14 kcal / mol.

3. Probe according to the claim 1, **characterized by** the fluorescent reporter (UV-F) that might be selected from the following tags: AttoRho6G, AttoRhol3, Atto520, AttoRho14, Atto495, Atto390, 6FAM.

4. Probe according to the claim 1, **characterized by** the fluorescence quencher (Q) that might be selected from the following quenchers: Eclipse, BBQ650, BHQ3.

5. Use of the probe (s) defined by claim 1-4 for detection of tick-borne pathogen (s) in simultaneous PCR reactions under same reaction conditions.

6. Use according to the claim 5, wherein the detection is performed in a PCR reaction with forward and reverse primers specified respectively by sequences:
5' TATTCCGCTATCCTCTCCCGG 3' and 5'CAATTATTGGGCGTAAAGGGCAT 3' for S_Ap probe or
5' GTCAGAGGTGAAATTCTTAGATTTGT 3' and 5' TGATTTCTCTCAAGTTGCTGAAGG 3' for S_Bb probe or
5' CCTGGGATTTCACCTCTGACTTAAAT 3' and 5' CAGCAGTGGGGAATATTGGACAA 3' for S_B1 probe or
5' GGTATCTAATCCTGTTTGCTCCCT 3' and 5' GGATTATTGGGCGTAAAGGGTGA 3' for S_Br probe or
5' ACTAGCTGTTGGGAAGTTCACT 3' and 5' GCACCAAATCATCTCTGACAAG 3' for S_Cb probe or
5' GAAAAGACAGAATAGGAAATGATTTTGTT 3' and 5' GCTAACCTCTACACAACTCTAGCC 3' for S_Mf probe or
5' TGTGTGAACGATGAAGGTCTTTAA 3' and 5' AAACTCCCTACCACACTCTAGATTA 3' for S_Mp probe or
5' CACTAAGAATTCCATCATCCCCTACTA 3' and 5' AGCAAGGAAGATAATGACGTTACTTG 3' for S_Rc probe.

## Patentansprüche

1. Schlauffenförmige UVEx sonde zum Nachweis von durch Zecken übertragenem pathogenem genetischem Material, das während der PCR Reaction durch *Taq*-DNA-Polymerase vom Typ "Hot Start" vergrößert wird, wobei (jede) Sonde **dadurch gekennzeichnet ist, dass** sie ein Oligonukleotid beliebiger Länge ist, das sich im Temperaturbereich von 4-24°C faltet und Sekundärstrukturen umfasst Schleifenbildung und unter Annahme der Grenzwerte ΔG (dG) aus dem Bereich -1.14 ≥ ΔG ≥ -8.36 kcal/mol, wobei der Abstand, in gerader Linie, zwischen dem Fluoreszenzemitter (UV-F) und dem Fluoreszenzlöscher (Q) in einer gewickelten Sonde 25 Nukleotide (nt) nicht überschreitet, während die Sonde(n) die folgenden Nukleotide umfasst(umfassen):
5' CAGGGTTAAGCCCTGGCGTTTCACCTT 3' bezeichnet als S_Ap und/oder
5' AAAGTTAGGGGCTCGAAGACGATCAGATACC 3' bezeichnet als S_Bb und/oder,
5' ACGCCCAGTAAATGCGAACAACGCTAGC 3' bezeichnet als S_B1 und/oder,
5' CAGTCTTGACCCAGAAGTTCGCCTTCGCC 3' bezeichnet als S_Br und/oder,
5' TAACGCGTTAAGTTCTCCGCCTGGGG 3' bezeichnet als S_Cb und/oder,
5' CTAACTATGTGCCAGCAGCCGCGGTAAT 3' bezeichnet als S_Mf und/oder,
5' TTTGGGAAGAATGACTTTAGCAGGTAACGGC 3' bezeichnet als S_Mp und/oder
5' AAAGCAATTCCGAGGTTAAGCCCCGAG 3' bezeichnet als S_Rc.

2. Die Sonde nach Anspruch 1 **dadurch gekennzeichnet, dass** der Grenzwert ΔG für die Sonde größer oder gleich -8.36 kcal/mol und kleiner oder gleich - 1.14 kcal/mol sein wird.

3. Die Sonde nach Anspruch 1 **dadurch gekennzeichnet, dass** der Fluoreszenzemitter (UV-F) unter den folgenden Tags ausgewählt werden kann: AttoRho6G, AttoRho13, Atto520, AttoRho14, Atto495, Atto390, 6FAM.

4. Die Sonde nach Anspruch 1 **dadurch gekennzeichnet, dass** der Fluoreszenzlöscher (Q) unter den folgenden Löschern ausgewählt werden kann: Eclipse, BBQ650, BHQ3.

5. Verwendung der Sonde definiert durch die Ansprüche 1 bis 4 zum Nachweis von durch Zecken übertragenen Pathogenen bei gleichzeitigen PCR unter den gleichen Reaktionsbedingungen.

6. Verwendung nach Anspruch 5 **dadurch gekennzeichnet** der Nachweis in einer PCR Reaction mit Vorwärts- und Rückwärtsprimern durchgeführt wird, die jeweils durch Sequenzen spezifiziert sind:
5' TATTCCGCTATCCTCTCCCGG 3' und 5'CAATTATTGGGCGTAAAGGGCAT 3' für S_Ap Sonde oder
5' GTCAGAGGTGAAATTCTTAGATTTGT 3' und 5'
TGATTTCTCTCAAGTTGCTGAAGG 3' für S_Bb Sonde oder
5' CCTGGGATTTCACCTCTGACTTAAAT 3' und 5'
CAGCAGTGGGGAATATTGGACAA 3' für S_B1 Sonde oder
5' GGTATCTAATCCTGTTTGCTCCCT 3' und 5' GGATTATTGGGCGTAAAGGGTGA 3' für S_Br Sonde oder
5'ACTAGCTGTTGGGAAGTTCACT3'und5'GCACCAAATCATCTCTGACAAG 3' für S_Cb Sonde oder
5' GAAAAGACAGAATAGGAAATGATTTTGTT 3' und 5'
GCTAACCTCTACACAACTCTAGCC 3' für S_Mf Sonde oder
5' TGTGTGAACGATGAAGGTCTTTAA 3' und 5'
AAACTCCCTACCACACTCTAGATTA 3' für S_Mp Sonde oder
5' CACTAAGAATTCCATCATCCCCTACTA 3' und 5'
AGCAAGGAAGATAATGACGTTACTTG 3' für S_Rc Sonde.

## Revendications

1. Sonde de type UVEx en boucle de type UVEx pour la détection du matériel génétique l'agent pathogène transmis par les tiques, qui est amplifiée pendant la réaction PCR par de type "Hot Start" Taq DNA polymérase, (chaque) sonde étant **caractérisée par le fait qu'**elle est un oligonucléotide de longueur quelconque, qui se replie à une plage de température comprise entre 4 et 24 °C et forme des structures secondaires et y compris les boucles) et que les valeurs limites ΔG (dG) sont comprises dans la plage -1,14 ≥ ΔG ≥ -8,36 kcal / mole, la distance, en ligne droite, entre le rapporteur fluorescent (UV-F) et l'extincteur de fluorescence (Q), dans une sonde enroulée, ne dépasse pas 25 nucléotides (nt), tandis que la sonde comprend(s) un ou plusieurs nucléotides suivants:
5' CAGGGTTAAGCCCTGGCGTTTCACCTT 3' nommés S_Ap et / ou
5' AAAGTTAGGGGCTCGAAGACGATCAGATACC 3' nommés S_Bb et / ou,
5' ACGCCCAGTAAATGCGAACAACGCTAGC 3' nommés S_B1 et / ou,
5' CAGTCTTGACCCAGAAGTTCGCCTTCGCC 3' nommés S_Br et / ou,
5' TAACGCGTTAAGTTCTCCGCCTGGGG 3' nommés S_Cb et / ou,
5' CTAACTATGTGCCAGCAGCCGCGGTAAT 3' nommés S_Mf et /ou,
5' TTTGGGAAGAATGACTTTAGCAGGTAACGGC 3' nommés S_Mp et / ou,
5' AAAGCAATTCCGAGGTTAAGCCCCGAG 3' nommés S_Rc.

2. La sonde, selon la revendication 1, **caractérisée en ce que** la valeur limite ΔG pour la sonde sera supérieure ou égale à -8,36 kcal / mol et sera inférieure ou égale à - 1,14 kcal / mol.

3. La sonde selon la revendication 1, **caractérisée par** le rapporteur fluorescent (UV-F) choisi parmi les marqueurs suivants : AttoRho6G, AttoRho13, Atto520, AttoRho14, Atto495, Atto390, 6FAM.

4. La sonde selon la revendication 1, **caractérisée par** l'extincteur de fluorescence (Q) pouvant être choisi parmi les extincteurs suivants : Éclipse, BBQ650, BHQ3.

5. Utilisation de la sonde défini dans les revendications 1 à 4 pour la détection de l'agent pathogène transmis par les tiques dans des réactions de PCR simultanées dans les mêmes conditions de réaction

6. Utilisation selon la revendication 5, **caractérisée** en ce la détection est effectuée dans une réaction PCR avec des amorces directe et inverse spécifiées respectivement par des séquences:
5' TATTCCGCTATCCTCTCCCGG 3' et 5'CAATTATTGGGCGTAAAGGGCAT 3' pour sonde S_Ap ou
5' GTCAGAGGTGAAATTCTTAGATTTGT 3' et 5' TGATTTCTCTCAAGTTGCTGAAGG 3' pour sonde S_Bb ou
5' CCTGGGATTTCACCTCTGACTTAAAT 3' et 5' CAGCAGTGGGGAATATTGGACAA 3' pour sonde S_B1 ou
5' GGTATCTAATCCTGTTTGCTCCCT 3' et 5' GGATTATTGGGCGTAAAGGGTGA 3' pour sonde S_Br ou
5'ACTAGCTGTTGGGAAGTTCACT3' et 5'GCACCAAATCATCTCTGACAAG 3' pour sonde S_Cb ou
5' GAAAAGACAGAATAGGAAATGATTTTGTT 3' et 5' GCTAACCTCTACACAACTCTAGCC 3' pour sonde S_Mf ou
5' TGTGTGAACGATGAAGGTCTTTAA 3' et 5' AAACTCCCTACCACACTCTAGATTA 3' pour sonde S_Mp ou
5' CACTAAGAATTCCATCATCCCCTACTA 3' et 5' AGCAAGGAAGATAATGACGTTACTTG 3' pour sonde S_Rc.
